(19) Europäisches Patentamt
European Patent Office
Office européen des brevets

(11) EP 0 862 547 B1

(12) # FASCICULE DE BREVET EUROPEEN

(45) Date de publication et mention de la délivrance du brevet:
**06.12.2000 Bulletin 2000/49**

(21) Numéro de dépôt: **96931125.7**

(22) Date de dépôt: **13.09.1996**

(51) Int Cl.[7]: **C07C 49/743**, C07C 45/78, C07C 69/007, C07C 69/738, C07C 59/82, A61K 7/48

(86) Numéro de dépôt international:
**PCT/FR96/01415**

(87) Numéro de publication internationale:
**WO 97/10196 (20.03.1997 Gazette 1997/13)**

(54) **PRODUITS EXTRAITS D'UNE PLANTE DU GENRE COMMIPHORA, EN PARTICULIER DE LA PLANTE COMMIPHORA MUKUL, ET EXTRAITS EN CONTENANT ET LEURS APPLICATIONS NOTAMMENT EN COSMETIQUE**

AUS EINER PFLANZE VON GESCHLECHT COMMIPHORA EXTRAJIERTE PRODUKTE, INSBESONDERE AUS DER PFLANZE COMMIPHORA MUKUL, UND DIESE ENTHALTENDE EXTRAKTEN SOWIE DEREN VERWENDUNG IN DER KOSMETIK

PRODUCTS EXTRACTED FROM A PLANT OF THE GENUS COMMIPHORA, PARTICULARLY THE COMMIPHORA MUKUL PLANT, EXTRACTS CONTAINING SAME AND APPLICATIONS THEREOF, FOR EXAMPLE IN COSMETICS

(84) Etats contractants désignés:
**DE ES FR GB IT**

(30) Priorité: **13.09.1995 FR 9510710**

(43) Date de publication de la demande:
**09.09.1998 Bulletin 1998/37**

(73) Titulaire: **PARFUMS CHRISTIAN DIOR**
**75008 Paris (FR)**

(72) Inventeurs:
- **ANDRE, Patrice**
  **F-45170 Neuvilles-aux-Bois (FR)**
- **LHERMITE, Stéphane**
  **F-45400 Semoy (FR)**
- **PELLICIER, Françoise**
  **F-45470 Loury (FR)**

(74) Mandataire: **Giraud, Françoise et al**
**Cabinet Beau de Loménie**
**158, rue de l'Université**
**75340 Paris Cedex 07 (FR)**

(56) Documents cités:
**EP-A- 0 513 671**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 862 547 B1

## Description

L'invention concerne l'utilisation en cosmétique d'extraits d'une plante du genre Commiphora, en particulier de la plante Commiphora mukul, notamment comme agent à activité anti-rides. Elle concerne également à titre de produits industriels nouveaux, deux produits particulièrement actifs isolés à partir de ces extraits ainsi que des dérivés de ces produits nouveaux.

On sait que la plante Commiphora mukul fait partie de la famille des Burséracées. Le Commiphora mukul est une plante originaire de l'Inde très utilisée en médecine traditionnelle de l'Inde, en médecine Ayurvédique. En particulier dans ces applications, on utilise une résine produite par Commiphora mukul appelée également Guggul. Dans ce traitement ayurvédique, on connaissait le traitement de l'obésité et des désordres lipidiques ainsi que des maladies rhumatismales.

Il est à noter que le terme "guggul" désigne à la fois la plante et la résine qu'elle produit. Par ailleurs, cette plante est un petit arbre ou un arbuste de 1,2 à 1,8 m de hauteur qui pousse essentiellement en Inde et l'incision de la plante permet de récolter la gomme-résine de manière courante.

Récemment, on a décrit dans les brevets US-A-4 847 071, US-A-4 847 069, US-A-4 946 671 et US-A-4 954 332, des compositions topiques pour la protection contre les radiations UV, contenant des absorbeurs de radicaux libres et un agent anti-inflammatoire. Parmi les nombreux agents anti-inflammatoires cités se trouve le Guggal ou extrait de Guggul.

D'autre part, il est également connu par le document EP-A-513 671 des compositions contenant un extrait lipophile total de la plante Commiphora mukul en particulier obtenu à partir de la résine d'écorce de Commiphora mukul comme ingrédient actif. Cet extrait a une forte proportion en Guggulstérones. Cette composition est décrite comme présentant une activité antiinflammatoire, immuno-modulatrice ou anti-androgène pour le traitement de l'acné et de l'hypertrophie bénigne de la prostate.

Il a été maintenant découvert de manière surprenante et inattendue que les extraits de plante du genre Commiphora, en particulier de la plante Commiphora mukul, présentent une activité anti-rides et peuvent ainsi être utilisés comme agents cosmétiques destinés à améliorer l'aspect de surface de la peau, en particulier pour diminuer la profondeur des rides et faire disparaître les ridules.

A partir de cette découverte, la demanderesse a fait des études systématiques complémentaires destinées à identifier des fractions particulièrement actives responsables de cette activité. Elle a constaté, en particulier, que ces fractions contenaient deux produits nouveaux particulièrement actifs dans l'activité concernée. Ces produits ont pu être isolés et totalement identifiés à partir d'extraits de la plante Commiphora mukul. Ils constituent donc des produits industriels nouveaux présentant une remarquable activité comme agents cosmétiques destinés à lutter contre les rides.

L'invention concerne également des dérivés des deux produits nouveaux isolés selon l'invention.

Ainsi, selon un premier aspect, l'invention concerne les produits répondant à la formule (I) :

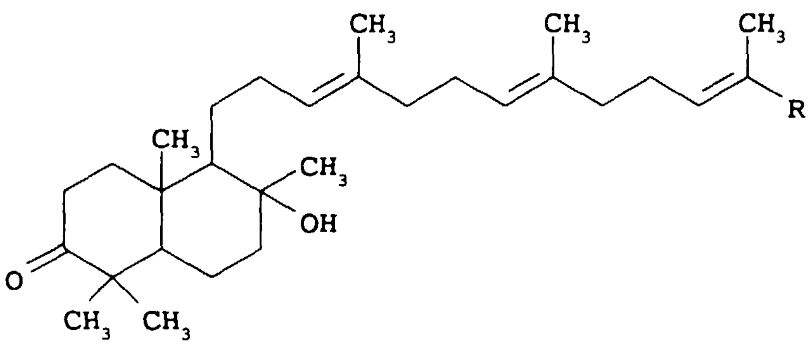

(I)

dans laquelle R représente :

a) un groupement $CH_2OH$,
b) un groupement COOH,

ainsi que leurs sels ou esters.

L'invention concerne tout particulièrement à titre de produits industriels nouveaux, les produits répondant à la formule (II) :

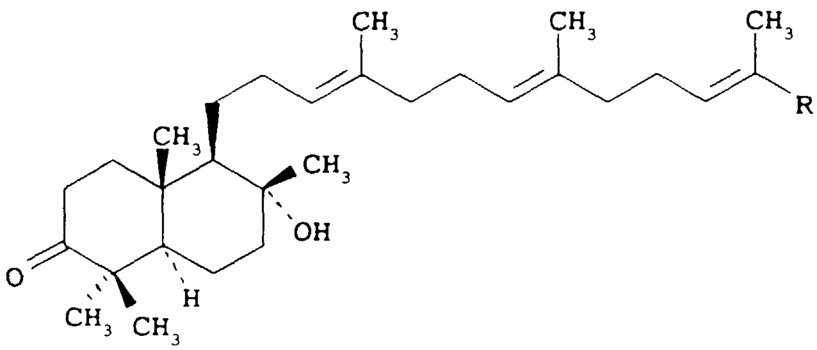

(II)

dans laquelle R représente :

a) un groupement $CH_2OH$, le produit étant désigné par la formule $II_a$,
b) un groupement COOH, le produit étant désigné par la formule $II_b$,
c) un groupement

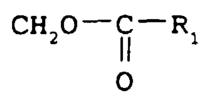

dans lequel $R_1$ représente un groupe alkyle linéaire ou ramifié comprenant de 1 à 6 atomes de carbone, en particulier le groupe méthyle,
d) un groupement COOM, où M désigne un métal alcalin, de préférence le sodium ou le potassium, ou un groupement ammonium ou amine quaternaire,
e) un groupement $COOM'_{0,5}$, où M' désigne un métal alcalino-terreux, de préférence le calcium,
f) un groupement $COOR_2$, où $R_2$ désigne un groupement alkyle linéaire ou ramifié comprenant de 1 à 6 atomes de carbone.

L'invention concerne tout particulièrement les deux produits industriels nouveaux désignés respectivement par $II_a$ et $II_b$ et répondant aux formules ci-dessous:

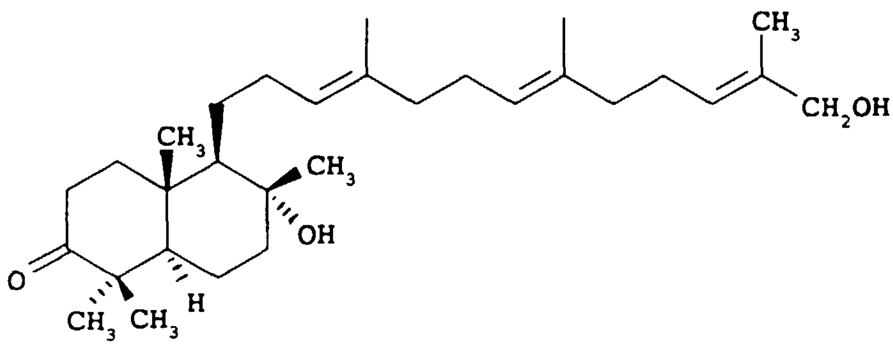

(IIa)

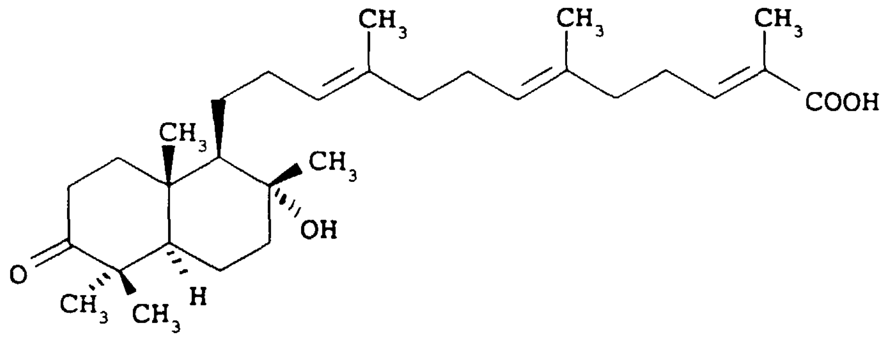

(IIb)

Les deux produits ont pu être isolés à partir de la plante Commiphora mukul et ont été complètement identifiés par différentes techniques analytiques comme cela ressort de la description qui suit.

Le produit répondant à la formule $II_a$, de formule brute $C_{30}H_{50}O_3$, sera désigné par "Commiphérol". Sa nomenclature est la suivante : (5R, 10S, 8R, 9R)-3-oxopolypoda-13E, 17E, 21 E-triène-8,30-diol.

Le dérivé acide répondant à la formule $II_b$, de formule brute $C_{30}H_{48}O_4$, sera désigné par "Commiphérine". Il s'agit de l'acide (5R, 10S, 8R, 9R)-8-hydroxy-3-oxopolypoda-13E, 17E, 21E-triène-30-oïque.

La nomenclature utilisée pour désigner les produits de formules $II_a$ et $II_b$ ci-dessus est basée sur le nom de l'hydrocarbure correspondant qui est un triterpène : l'α-polypodatétraène, bien connu dans la littérature, par exemple dans la publication de Yoko Arai et al., Tetrahedron Letters, (1992) 33 (10) 1325-8, relative à la plante Polypodiodes formosane. La numération des atomes de carbone répond à celle indiquée ci-dessous :

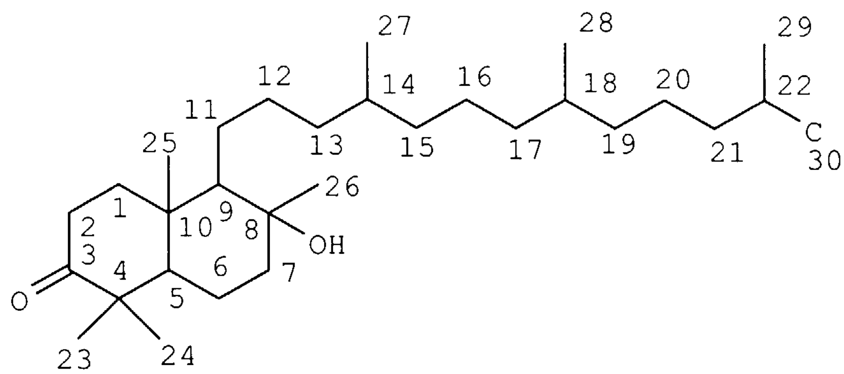

Il est à noter que divers dérivés triterpéniques possédant le squelette carboné polypodane ont été identifiés dans d'autres plantes, en particulier dans des fougères du genre des Polypodiacées telles que Polypodium vulgare, P. fauriei et P. virginianum (Y. Arai et al., Phytochemistry, (1991) 30 (10) 3369-3377 ; K. Shiojima et al., Tetrahedron Lett., (1983) 24 5733), des Aspidiacées (M. Nishizawa et al., J. Chem. Soc., Chem. Commun. (1984) N°7, 467-8) et des Cheiropleuriacées (R. Karnaya et al., Chem. Pharm. Bull. (1990) 38 (8) 2130-2).

Selon un second aspect, l'invention concerne également des procédés de préparation des deux produits $II_a$ et $II_b$ à partir de la plante Commiphora mukul ainsi que des dérivés d'acylation du produit $II_a$, en particulier le produit d'acétylation et des sels et esters du produit $II_b$.

Différents procédés peuvent être utilisés pour isoler les produits de formules $II_a$ et $II_b$ à partir de la plante Commiphora mukul.

Dans ces procédés, on part avantageusement de la résine de Commiphora mukul que l'on soumet à différentes étapes d'extraction et de fractionnement successives.

On soumet ainsi de façon particulièrement avantageuse la résine de Commiphora Mukul à une première étape dite d'extraction par un solvant ou un mélange de solvants, puis on soumet ensuite l'extrait à différentes étapes de séparation destinées à isoler une fraction particulièrement active contenant au moins un des produits de l'invention.

Pour réaliser la première étape d'extraction, on peut utiliser une large gamme de solvants de polarités très différentes.

A titre d'exemples de solvants utilisables pour réaliser cette étape, on citera, par ordre de polarité croissante :

- l'éther de pétrole qui permet d'extraire 16 % en poids de la résine brute,
- le dichlorométhane qui permet d'extraire 26 % en poids de la résine brute,
- l'acétate d'éthyle qui permet d'extraire 30,5 % en poids de la résine brute,
- l'éthanol qui permet d'extraire 26,5 % en poids de la résine brute.

La figure 1 représente schématiquement différents protocoles permettant de préparer les produits de formules $II_a$ et $II_b$ à partir d'un extrait de la résine de Commiphora mukul selon l'invention.
Selon le schéma de la figure 1, on prépare à partir de la résine de Commiphora mukul un premier extrait selon l'invention, dénommé extrait G.
Cet extrait G est obtenu par extraction par l'éthanol à 96 % à 45°C de la résine après broyat des agrégats.
Selon une première étape, l'extrait G est ensuite soumis à une succession de fractionnements par chromatographie liquide haute performance. Chaque fraction est testée pour son activité lipogénétique sur des fibroblastes en culture, selon la méthode exposée plus loin. Ces différents fractionnements aboutissent à l'obtention d'une fraction active, FIIB, dont on repère les pics caractéristiques sur le chromatogramme.
Plus précisément, selon cette première étape, l'extrait G sera soumis dans un premier temps à un protocole B destiné à isoler les fractions les plus actives permettant de séparer par chromatographic liquide haute performance l'extrait G en trois fractions F représentant 92,5 % de l'extrait G, FIII représentant 4,5 % de cet extrait et FIV représentant 3 %. La fraction FIII a pu être identifiée comme constituée essentiellement des stérols et la fraction FIV se compose essentiellement des produits de rinçage de la colonne chromatographique par le dichlorométhane.
La fraction F est ensuite soumise au protocole C de séparation par chromatographie liquide, qui conduit à deux fractions dénommées respectivement FI et FII. On élimine la fraction F I qui est sensiblement inactive. Elle représente 21,5 % de F, et est constituée essentiellement des stérones (Z- et E-guggulstérone).
La fraction FII, qui présente une activité lipogénétique, est à son tour soumise à un fractionnement par chromatographie liquide permettant de la séparer en trois fractions : FIIA représentant 19,5 % de l'extrait G, FIIB représentant 20 % de l'extrait G et FIIC représentant 31,5 % de l'extrait G. De ces trois fractions, la fraction FIIB est la plus active. On repère sur le chromatogramme la position des pics correspondants.
Il est donc ensuite possible, dans une deuxième étape, d'obtenir par fractionnement selon le protocole E directement à partir de l'extrait G une fraction active, dénommée FIIB1, correspondant aux pics de la fraction FIIB.
Ensuite, les deux produits de formules $II_a$ et $II_b$ peuvent être séparés à partir de l'extrait FIIB1 par chromatographie liquide haute performance préparative.
Les deux produits $II_a$ et $II_b$, dont l'activité s'est avérée sensiblement équivalente, ont été parfaitement purifiés et ont pu être isolés par chromatographie liquide en utilisant les conditions suivantes :

- Colonne RP 18 Lichrospher 5 $\mu$m 125 x 4 mm
- Mélanges : Eau + 0,1 % $CF_3$ COOH
  Acétonitrile
- Détection UV : $\lambda$ = 210 nm.

Le produit de formule $II_a$ peut ensuite être soumis à une étape classique d'acylation, en particulier d'acétylation, pour préparer les dérivés acylés correspondants, en particulier le dérivé acétylé.
Le produit $II_b$ peut être aisément transformé en l'un de ses sels décrits ci-dessus par neutralisation de la fonction acide en bout de chaîne par une base correspondante.
On pourra de même aisément accéder aux produits d'estérification du produit $II_b$ en le faisant réagir classiquement avec un alcool léger.
Ces réactions, destinées à acyler le produit $II_a$ ou à estérifier ou salifier le produit $II_b$, peuvent également être réalisées directement sur un mélange contenant les deux produits actifs $II_a$ et $II_b$, en particulier sur le mélange FIIB1 décrit ci-dessus.
Par exemple, on pourra effectuer l'acétylation de $II_a$ par l'anhydride acétique en traitant l'extrait FIIB1 de la façon suivante : on dissout FIIB1 dans du dichlorométhane (1 volume), on ajoute 1 volume de pyridine puis 1,2 équivalent d'anhydride acétique pour 1 équivalent de FIIB1 et on laisse la réaction se faire à température ambiante durant toute une nuit.
La demanderesse a constaté de façon tout à fait surprenante que les extraits de la plante Commiphora mukul, en particulier les extraits particulièrement riches en produits répondant à la formule $II_a$ ou $II_b$, présentaient une activité stimulatrice de la lipogénèse interne aux fibroblastes. Ceci se traduit par une augmentation de volume cellulaire des fibroblastes conduisant à un meilleur contact avec le réseau protéique extra-cellulaire. Le derme se trouve ainsi tonifié, ce qui permet de diminuer la profondeur des rides et des ridules, et, en conséquence, de les rendre moins apparentes. Cette activité a donc permis à la demanderesse de proposer une solution particulièrement originale pour améliorer l'aspect de surface de la peau.

Ainsi, selon un troisième aspect, l'invention concerne l'utilisation d'au moins un extrait de plante du genre Commiphora, en particulier de la plante Commiphora mukul, comme agent cosmétique destiné à modifier la surface de la peau, en lui conférant un aspect plus lisse par l'atténuation de la profondeur des rides et des ridules.

On pourra utiliser dans cette application différents extraits de la plante Commiphora mukul, en particulier des extraits riches en composés répondant aux formules I, II, $II_a$ ou $II_b$, ou en leur mélange ou en dérivés de ces produits, tels que définis précédemment.

On pourra, selon une première variante, utiliser comme extrait de la plante Commiphora mukul la résine-gomme dite Guggul.

Selon une autre variante, on utilise comme extrait de la plante Commiphora mukul un extrait obtenu après broyat des agrégats de la résine, puis extraction par un solvant. Comme on l'a vu précédemment, une large gamme de solvants pourra être utilisée à cet effet.

Toutefois, en se référant à la classification des solvants par polarité telle que publiée en particulier par Veronika R. Meyer dans Practical High - performance Liquid Chromatography (1988), John Wiley & Sons, p. 120-121, on choisira, de préférence, les solvants dont le paramètre de polarité p' est inférieur à 5,5 et de préférence compris entre 0,1 et 4,5.

L'extrait ci-dessus est avantageusement obtenu par extraction par un solvant organique ou un mélange de solvants organiques choisis dans le groupe constitué par : le n-pentane, le n-hexane, l'éther de pétrole, le cyclohexane, le n-décane, le dichlorométhane, l'isopropanol, le n-propanol, le chloroforme, l'éthanol, l'acétate d'éthyle, l'acétone et le méthanol.

Selon d'autres variantes de l'invention, l'extrait pourra être constitué de différents produits présentant le caractère commun d'être enrichis en au moins l'un des produits de formule I ou II, en particulier de formule $II_a$ ou $II_b$, obtenus à partir de l'extrait décrit ci-dessus.

Comme cela est clairement illustré dans les exemples donnés en référence au schéma de la figure 1, les produits enrichis en produit(s) de l'invention peuvent être obtenus par exemple à partir de l'extrait G, en soumettant cet extrait à différentes étapes successives de séparation, notamment par Chromatographie Liquide Haute Performance ou par extraction au gaz carbonique supercritique.

Selon une variante particulièrement avantageuse de l'invention, on utilisera au moins l'un des produits de formule I ou II, en particulier de formule $II_a$ ou $II_b$, comme agent cosmétique pour modifier la surface de la peau comme indiqué précédemment.

Selon un quatrième aspect, l'invention concerne également une composition, en particulier destinée à un usage cosmétique, caractérisée en ce qu'elle contient l'un au moins des produits de formule I ou II, en particulier $II_a$ ou $II_b$ ou des extraits de plante contenant au moins l'un de ces produits, en particulier des extraits de plante du genre Commiphora, encore en particulier de la plante Commiphora mukul, de préférence en combinaison avec un excipient ou support acceptable, en particulier cosmétiquement acceptable.

Avantageusement, cette composition contient de 0,001 à 1 % en poids de l'un au moins des produits de formule (I) ou (II), en particulier ($II_a$) ou ($II_b$), de préférence de 0,01 à 0,1 %.

Ou encore cette composition contient très avantageusement de 0,005 à 5 % en poids, de préférence de 0,05 à 1 % en poids d'un extrait de Commiphora mukul contenant au moins l'un des produits précités, en particulier un extrait de résine de cette plante.

Les compositions cosmétiques de l'invention peuvent être sous différentes formes, en particulier sous forme de solutions, de lait, de gel, de crème.

Selon une variante de l'invention, la composition cosmétique contient, en outre, une quantité cosmétiquement efficace d'un produit agissant sur la synthèse de la fibronectine et/ou sur la synthèse du collagène.

A titre d'exemple de produit agissant sur la synthèse de la fibronectine, on citera les galactolipides, en particulier les galactosylglycérides dont l'utilisation est décrite dans la demande de brevet français déposée le 15 février 1995 et publiée sous le N°2 730 409.

A titre d'exemple de produit agissant sur la synthèse du collagène, on citera la vitamine C.

L'invention concerne également un procédé de traitement cosmétique destiné à modifier la surface de la peau en lui conférant un aspect plus lisse par l'atténuation de la profondeur des rides et des ridules, caractérisé en ce que l'on applique sur les zones de la peau à traiter, en particulier sur le visage, une quantité efficace d'un produit ou d'un extrait de plante en contenant, pour obtenir ladite modification de surface, ledit produit ou ledit extrait étant de préférence incorporé dans un excipient cosmétiquement acceptable.

Selon une variante de réalisation de ce procédé de traitement cosmétique, l'extrait de plante précité est un extrait de la plante Commiphora mukul.

D'autres caractéristiques et avantages de l'invention apparaîtront à la lecture des exemples qui suivent donnés à titre purement illustratif de l'invention.

## EXEMPLES

### Exemple 1 : Préparation de l'extrait G selon l'invention

On prépare cet extrait par extraction de la résine de Commiphora mukul après broyat des agrégats.
L'extrait est réalisé par l'éthanol à 96 % à 45°C de la façon suivante : on introduit 10 g de résine et 200 ml d'éthanol dans un ballon de 500 ml muni d'un réfrigérant et d'un agitateur et dont le chauffage est assuré par une plaque chauffante.
L'agitation et l'extraction durent 2 h minimum, mais il est conseillé de laisser 4 à 5 h pour un meilleur rendement.
Une filtration est faite après extraction suivie d'une évaporation sous vide.
Le rendement d'extraction est d'environ 25 % en poids.

### Exemple 2 : Préparation de la fraction F II B1 à partir de l'extrait G

Cet exemple est donné en référence au schéma de la figure 1. On donne, dans le tableau 1 ci-dessous, des précisions sur les protocoles B, C, D et E en ce qui concerne la nature et les caractéristiques des colonnes de chromatographie utilisées ainsi que le type de détecteur et la nature des éluants utilisés.

TABLEAU 1

| Commiphora mukul | | | | | |
|---|---|---|---|---|---|
| | Produit à purifier | Colonne | Détecteur | Eluants | Fractions obtenues |
| Protocole B | extrait G | kromasil 100 C18 150 x 21,7 mm 13 µm | 220 nm | méthanol | F, FIII, FIV |
| Protocole C | fraction F | kromasil 100 C18 150 x 21,7 mm 13 µm | 210 nm | gradient méthanol eau | FI, FII |
| Protocole D | fraction FII | kromasil 100 C18 150 x 21,7 mm 13 µm | 210 nm | gradient méthanol eau | FIIA, FIIB, FIIC |
| Protocole E | extrait G | kromasil 100 C18 150 x 21,7 mm 13 µm | détecteur à diffusion de lumière (DEDL) | gradient méthanol eau | FIIB1 |

### Exemple 3 : Obtention des produits $II_a$ et $II_b$ à partir de la fraction FIIB1

Les deux produits $II_a$ et $II_b$ ont été séparés par chromatographie liquide préparative, et purification sur colonne C18 en élution isocratique par la technique de recyclage. Une détection à 210 nm permet de visualiser les produits $II_a$ et $II_b$.
Les deux produits de formules $II_a$ et $II_b$ ont été totalement identifiés par spectrométrie de masse qui a permis de vérifier les formules brutes établies par RMN.
Les résultats obtenus par RMN du carbone pour les deux produits $II_a$ et $II_b$ sont donnés dans les tableaux 2 et 3 ci-dessous :

TABLEAU 2

| Déplacements chimiques des différents groupements carbonés du produit $II_a$ | | | | | |
|---|---|---|---|---|---|
| Atomes N° | $\delta$ $^{13}C$ ppm | Multiplicité | Atomes N° | $\delta$ $^{13}C$ ppm | Multiplicité |
| 1 | 38,35 | $CH_2$ | 16 | 26,33 | $CH_2$ |
| 2 | 34,02 | $CH_2$ | 17 | 124,54 | CH |
| 3 | 217,16 | Cquat | 18 | 134,68 | Cquat |

TABLEAU 2 (suite)

| Déplacements chimiques des différents groupements carbonés du produit $II_a$ | | | | | |
|---|---|---|---|---|---|
| Atomes N• | δ $^{13}C$ ppm | Multiplicité | Atomes N• | δ $^{13}C$ ppm | Multiplicité |
| 4 | 47,57 | Cquat | 19 | 39,67 | $CH_2$ |
| 5 | 55,18 | CH | 20 | 26,16 | $CH_2$ |
| 6 | 21,41 | $CH_2$ | 21 | 126,04 | CH |
| 7 | 43,80 | $CH_2$ | 22 | 134,71 | Cquat |
| 8 | 73,82 | Cquat | 23 | 21,41 | $CH_3$ |
| 9 | 60,36 | CH | 24 | 26,33 | $CH_3$ |
| 10 | 39,39 | Cquat | 25 | 14,89 | $CH_3$ |
| 11 | 25,80 | $CH_2$ | 26 | 23,62 | $CH_3$ |
| 12 | 31,20 | $CH_2$ | 27 | 16,06 | $CH_3$ |
| 13 | 124,83 | CH | 28 | 16,27 | $CH_3$ |
| 14 | 135,47 | Cquat | 29 | 13,77 | $CH_3$ |
| 15 | 39,34 | $CH_2$ | 30 | 68,97 | $CH_2$ |

TABLEAU 3

| Déplacements chimiques des différents groupements carbonés du produit $II_b$ | | | | | |
|---|---|---|---|---|---|
| Atomes N• | δ $^{13}C$ ppm | Multiplicité | Atomes N• | δ $^{13}C$ ppm | Multiplicité |
| 1 | 38,28 | $CH_2$ | 16 | 26,28 | $CH_2$ |
| 2 | 34,02 | $CH_2$ | 17 | 125,47 | CH |
| 3 | 217,10 | Cquat | 18 | 134,55 | Cquat |
| 4 | 47,57 | Cquat | 19 | 38,03 | $CH_2$ |
| 5 | 55,18 | CH | 20 | 25,92 | $CH_2$ |
| 6 | 21,35 | $CH_2$ | 21 | 143,82 | CH |
| 7 | 43,59 | $CH_2$ | 22 | 133,43 | Cquat |
| 8 | 74,54 | Cquat | 23 | 21,41 | $CH_3$ |
| 9 | 60,48 | CH | 24 | 26,33 | $CH_3$ |
| 10 | 39,39 | Cquat | 25 | 14,89 | $CH_3$ |
| 11 | 25,85 | $CH_2$ | 26 | 23,54 | $CH_3$ |
| 12 | 31,44 | $CH_2$ | 27 | 16,09 | $CH_3$ |
| 13 | 125,15 | CH | 28 | 15,88 | $CH_3$ |
| 14 | 134,71 | Cquat | 29 | 12,27 | $CH_3$ |
| 15 | 39,38 | $CH_2$ | 30 | 171,14 | Cquat |

Exemple 4 : Extraction par $CO_2$ supercritique

L'extraction est réalisée sur environ 240 g de résine brute broyée, en deux étapes de la façon suivante :

- Etape 1 :

Cette étape est réalisée dans un appareillage classique, avec du $CO_2$ pur à 150 bars ct 40°C.

La consommation de $CO_2$ est de 2 000 kg de $CO_2$ pour 24 kg de résine à extraire.
Le rendement d'extraction est d'environ 12,50 % par rapport à la charge de départ. L'extrait obtenu est éliminé.

Etape 2:

Cette étape est réalisée dans le même appareillage, avec un mélange contenant en poids 98 % de $CO_2$ à 98 % et 2 % d'éthanol.
On utilise 1 000 kg de $CO_2$ pour 24 kg de résine brute (soit 18 kg de $CO_2$ pour 240 g de résine).
Le rendement d'extraction est d'environ 10 % par rapport à la charge de départ. On obtient un extrait dit extrait SFE (supercritical fluid extraction) enrichi en molécules $II_a$ et $II_b$ avec un facteur de concentration de 6 à 8 par rapport à la résine brute.

Exemple 5 : Mise en évidence de l'activité des extraits et produits selon l'invention

1. Culture

Les cellules utilisées sont des 3T3 F442A qui constituent une lignée de pré-adipocytes murins sélectionnés pour leur capacité à se convertir en adipocytes si les conditions de culture le permettent, conformément à la méthode de Green, H & Kehinde, C, Cell 1 (1974) 113.
Cette lignée constitue en effet un modèle d'étude de la différenciation adipocytaire in vitro.
Les 3T3 F442A en monocouche lors de la phase de multiplication présentent la morphologie et les caractéristiques enzymatiques de fibroblastes.
Les cellules confluentes dans un premier temps cessent de se diviser pour entrer dans leur phase de différenciation précoce. Cette différenciation conduit à la formation de colonies de cellules qui subissent la conversion adipocytaire.
Cette différenciation s'accompagne de changements dans la biosynthèse de plusieurs protéines et d'une augmentation d'activités enzymatiques différentes (Acetyl CoA carboxylase, ATP citrate lyase, Fatty acid synthétase, phosphoenolpyruvate kinase, glycéro-3-phosphate deshydrogénase dénommé $G_3$PDH).
On s'est attaché à mesurer l'expression de deux marqueurs de différenciation, la glycéro-3-phosphate deshydrogénase ($G_3$PDH) d'une part et l'AMP cyclique (AMPc) d'autre part.
Il est rappelé à ce propos que l'enzyme $G_3$PDH permet la formation dans le fibroblaste du glycérol 3-phosphate, molécule impliquée par la suite dans la synthèse des lipides intracellulaires (triglycérides). Ainsi, l'augmentation de l'activité de la $G_3$PDH est directement reliée au renforcement de cette synthèse.
Par ailleurs, il est connu que la quantité d'AMPc, médiateur intracellulaire, augmente lors de la réaction de la lipolyse intracellulaire. L'AMPc formée dans la cellule est ensuite excrétée par celle-ci dans le milieu extra-cellulaire . Ainsi, la diminution du taux d'AMPc dans le milieu de culture traduit une diminution de la dégradation des triglycérides, donc une accumulation intracellulaire de ces lipides.
La résine de Commiphora Mukul (extrait G), la fraction FIIB1 ainsi que les produits $II_a$ et $II_b$ ont donc été étudiés avec ces deux marqueurs de différenciation.
Les fibroblastes sont ensemencées au fond des boîtes de Pétri de 35 mm de diamètre avec du milieu de culture "Dulbecco's Modified Eagle Médium" (DMEM) en présence de 5 % de sérum de veau (SV) et 5 % de sérum de veau foetal (SVF). Chaque essai est fait en triple.
Pendant la phase de traitement, le milieu est constitué de DMEM + 10 % de sérum de veau foetal.
Le produit ou l'extrait selon l'invention est mis en solution dans de l'éthanol et utilisé sur les cultures à la concentration finale de 5 µg/ml.
Les opérations de culture se déroulent de la manière suivante :

- Au jour J = 0 : ensemencement dans du milieu DMEM + 5 % SV, 5 % SVF
- Au jour J + 2 : changement de milieu
- Au jour J + 5 : traitement par la résine de Commiphora mukul (Extrait G), FIIB1 ou $II_a$ ou $II_b$ à 5 µg/ml dans du milieu DMEM, 10 % SVF
- Au jour J + 6 : dosage AMPc dans du milieu de culture
- Aux jours J + 7 et J + 9 : traitement identique à celui effectué à J + 5
- Au jour J + 12 : broyage des cellules pour dosage $G_3$PDH.

2. Dosage de l'adénosine 3-5-monophosphate cyclique (AMPc)

Le dosage de l'AMPc réalisé par Radio Immuno Assay dit RIA (Kit Immunotech, société française, référence

1117) est basé sur le principe de la compétition antigène-anticorps. Les échantillons et les standards sont incubés en présence d'AMPc radiomarqué à l'iode 125 dans des tubes où sont préalablement fixés des anticorps anti AMPc.

Après incubation, le contenu des tubes est aspiré et on compte la radioactivité résiduelle à l'aide d'un compteur gamma. Une courbe standard est préparée avec 6 concentrations connues d'AMPc et on définit la concentration des échantillons grâce à cette courbe étalon.

L'AMPc étant produit et excrété par les cellules, on mesure donc l'AMPc contenu dans le milieu de culture. Plus précisément, on mesure la quantité d'AMPc excrétée dans le milieu de culture en 24 heures.

3. <u>Détermination de l'activité glycéro-3-phosphate déshydrogénase ($G_3PDH$)</u>

La monocouche cellulaire est récupérée par grattage et vigoureusement homogénéisée dans du tampon TRIS-HCl (25 mM, pH 7,4) 1 mmole EDTA à 4°C. Le dosage de l'activité $G_3PDH$ est déterminé sur le surnageant du broyat cellulaire aussitôt après centrifugation.

La $G_3PDH$ catalyse la réaction suivante :

**DHAP** **+ NADH --------> glycérol-3-phosphate + NAD** (enzyme : **$G_3PDH$**)
**dihydroxyacétone phosphate**

On mesure en spectrophotométrie à 340 nm la transformation en fonction du temps du coenzyme NADH (nicotinamide-adénine-dinucléotide hydrogéné) en NAD, ce qui traduit la vitesse de la réaction enzymatique, et donc l'activité de l'enzyme $G_3PDH$.

On peut calculer une différence d'absorption (ΔAbs)/min qui correspond à la vitesse initiale de la réaction enzymatique.

Les résultats sont exprimés en activité spécifique soit en nmoles de NADH transformées /min/ mg de protéines cellulaires (le taux de protéines cellulaires total est évalué par la méthode du BCA - PIERCE : protein assay Reagent.).

4.1. <u>Activité $G_3PDH$</u>

Les résultats expérimentaux de l'évaluation de l'activité de l'enzyme $G_3PDH$ figurent au tableau 4 ci-dessous.

L'activité $A_1$ des produits selon l'invention sur la stimulation de l'activité de cette enzyme est calculée selon la formule ci-dessous :

$$A_1 = \frac{V_p - V_t}{V_t} \times 100$$

dans laquelle :
$V_p$ est la valeur moyenne entre les trois essais de la vitesse de transformation de la NADH exprimée en nmoles/mn/mg de protéines cellulaires dans les cultures traitées par les produits selon l'invention
$V_t$ est la valeur moyenne de cette vitesse dans les cultures témoins.

TABLEAU 4

| Cultures | Vitesse de transformation de la NADH nmoles/mn/mg de protéines | Activité $A_1$ % |
|---|---|---|
| Témoins | 49,6 ± 3,39 | 0 |
| Extrait G | 156 ± 43 | 215 |
| Fraction FIIB1 | 242 ± 42 | 394 |
| $II_a$ | 222 ± 88 | 348 |
| $II_b$ | 352 ± 62 | 610 |

Il ressort donc très clairement du tableau 4 que les produits selon l'invention, qu'il s'agisse de l'extrait G, de la fraction FIIB1 ou des produits $II_a$ ou $II_b$, augmentent très fortement l'activité de l'enzyme $G_3PDH$ dans les cultures

de fibroblastes, par rapport à l'activité de cette enzyme dans les cultures témoins. On observe en effet que l'activité de cette enzyme est multipliée par 3 par l'action de l'extrait G, et multipliée par 7 par l'action du composé $II_b$. Ainsi, il est démontré que les produits selon l'invention contribuent à augmenter de manière importante la synthèse de triglycérides intracellulaires.

4.2 Dosage d'AMPc

La quantité d'AMPc excrétée en 24 heures dans le milieu des différentes cultures réalisées figure au tableau 5. Elle est exprimée en nmoles/litre de milieu. L'activité $A_2$ des produits selon l'invention sur l'excrétion de l'AMPc par les cellules, en rapport direct avec la réaction de lipolyse intracellulaire, est calculée selon la formule ci-dessous :

$$A_2 = \frac{q_p - q_t}{q_t} \times 100$$

dans laquelle :
$q_p$ représente la quantité moyenne entre les trois essais d'AMPc excrétée dans le milieu des cultures traitées par les produits selon l'invention, exprimée en nmoles/litre/24 heures
$q_t$ représente cette quantité moyenne dans le cas des cultures témoins.

TABLEAU 5

| Cultures | AMPc excrétée nmoles/litre/24 heures | $A_2$ % |
|---|---|---|
| Témoins | 66 ± 3,5 | 0 |
| Extrait G | 56,8 ± 3,8 | - 13,9 |
| FIIB1 | 42 ± 2 | - 36,4 |

Ces résultats figurant au tableau 5 montrent que la quantité d'AMPc excrétée dans le milieu de culture est plus faible dans le cas des cultures traitées par les produits de l'invention que dans le cas des cultures témoins.
Ainsi, dans les cellules de cultures traitées, il apparaît que la lipolyse est très nettement inférieure à celle qui se produit dans les cellules des cultures témoins.
En conclusion, ces essais mettent clairement en évidence que les produits selon l'invention agissent par deux voies complémentaires pour augmenter la quantité de lipides intracellulaires, d'une part en favorisant leur synthèse, ce qui est démontré par l'augmentation de l'activité de l'enzyme $G_3$PDH, et d'autre part en limitant leur dégradation, ce qui est démontré par la diminution du taux d'AMPc dans les cultures traitées.
On observera en outre que les deux molécules $II_a$ et $II_b$ donnent les résultats les plus élevés apparaissant effectivement comme étant les supports de l'activité lipogénétique des extraits et fractions selon l'invention.
Ainsi, les produits selon l'invention accélèrent la différenciation adipocytaire des fibroblastes. De plus, en raison de l'accumulation des lipides, ces cellules augmentent leur volume, permettant ainsi un meilleur contact avec le réseau protéique extra-cellulaire. Le derme se trouve alors consolidé. Cette tonicité du derme entraine alors une diminution de la profondeur des rides et ridules et confère à la surface de la peau un aspect plus lisse.

Exemple 6 : Crème anti-rides

On prépare une crème anti-rides par mélange des constituants ci-dessous donnés avec leurs pourcentages en poids par rapport à la composition finale en suivant le protocole de préparation suivant :
On prépare un mélange A constitué de :

| | |
|---|---|
| Brij 72® | 0,8 |
| Brij 721® | 2,2 |
| Tegin 90® | 1,7 |
| Alcool stéarylique | 1,8 |
| Stéarine | 3,0 |
| Huile de silicone (Fluid 200®) | 0,20 |
| Squalane | 10,0 |
| Miglyol 812® | 10,0 |

(suite)

| | |
|---|---|
| Acétate de D,L-α-tocophérol | 0,2 |
| Phénonip | 0,5 |
| Extrait supercritique SFE de l'exemple 4 | 0,5 |

On ajoute le mélange B constitué de :

| B: | |
|---|---|
| - Glycérine | 5,00 |
| - Eau | 58,53 |
| Carbopol 940 | 0,20 |

puis C, D et E respectivement constitués de :

| | | |
|---|---|---|
| C | Soude 10 % | 0,07 |
| D | Protéines de blé | 5,00 |
| E | Parfum | 0,30 |

Exemple 7 : Gel pour le contour des yeux à activité anti-rides

On prépare une composition par mélange des composants notés A, B, C, D et E ci-dessous dont les constituants sont donnés avec leurs pourcentages en poids par rapport à la composition finale.

| A: | |
|---|---|
| - Carbopol 1342® | 0,40 |
| - Eau | 83,20 |
| B : Solution de soude 10 % | 0,40 |

| C : | |
|---|---|
| - Produit $II_a$ selon l'invention | 0,1 |
| - Miglyol 829® | 10,00 |
| - Phénonip | 0,50 |
| - Acétate de D,L-α-tocophérol | 0,20 |
| D : Protéines de blé | 5,00 |
| E : Parfum | 0,20 |

**Revendications**

1. Produit répondant à la formule (I) :

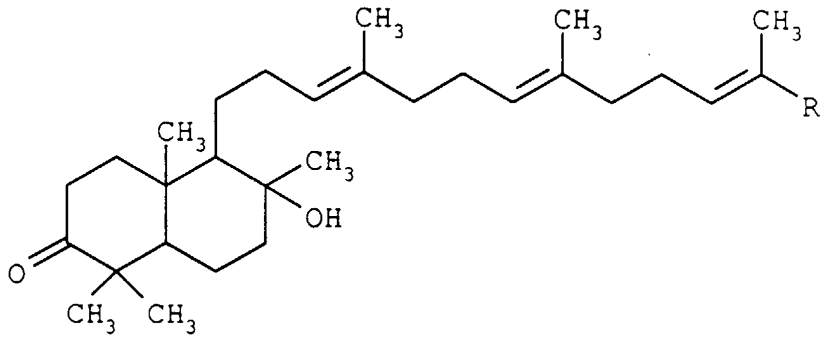

(I)

dans laquelle R représente :

a) un groupement $CH_2OH$,
b) un groupement COOH,

ainsi que ses sels ou esters.

**2.** Produit selon la revendication 1 répondant à la formule (II) :

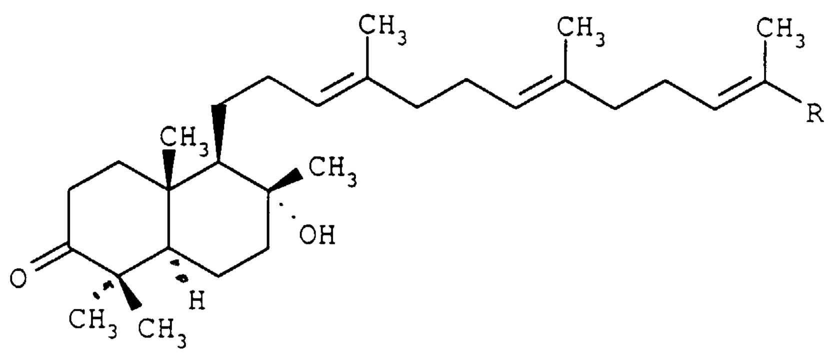

(II)

dans laquelle R représente :

a) un groupement $CH_2OH$, le produit étant désigné par la formule $II_a$,
b) un groupement COOH, le produit étant désigné par la formule $II_b$,
c) un groupement

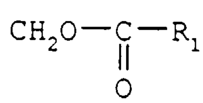

dans lequel $R_1$ représente un groupe alkyle linéaire ou ramifié comprenant de 1 à 6 atomes de carbone, en particulier le groupe méthyle,
d) un groupement COOM, où M désigne un métal alcalin, de préférence le sodium ou le potassium, ou un groupement ammonium ou amine quaternaire,
e) un groupement $COOM'_{0,5}$, où M' désigne un métal alcalino-terreux, de préférence le calcium,
f) un groupement $COOR_2$, où $R_2$ désigne un groupement alkyle linéaire ou ramifié comprenant de 1 à 6 atomes de carbone.

**3.** Produit selon la revendication 2, caractérisé en ce qu'il répond à la formule ($II_a$)

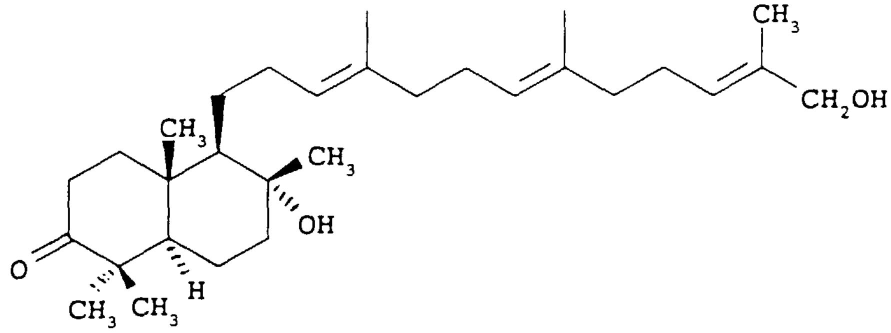

(IIa)

4. Produit selon la revendication 2, caractérisé en ce qu'il répond à la formule ($II_b$)

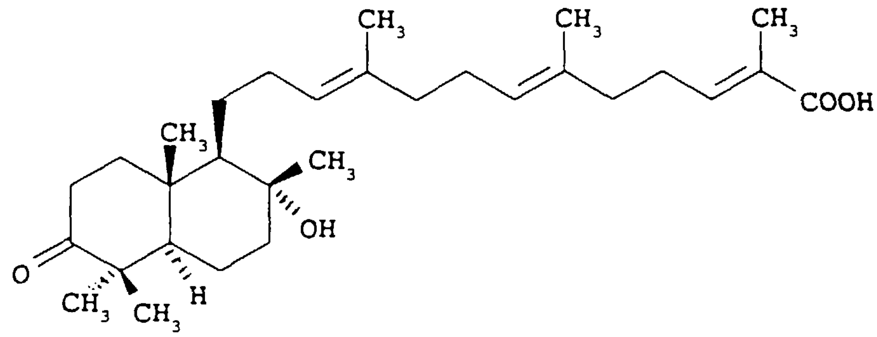

(IIb)

5. Procédé de préparation d'un produit tel que défini dans l'une des revendications 1 à 4, caractérisé en ce qu'il consiste à traiter, par extraction par un solvant organique, de la résine de Commiphora mukul pour la préparation d'un extrait dit extrait G et à soumettre ledit extrait G à au moins une étape de fractionnement pour isoler une fraction constituée majoritairement d'un produit répondant à la formule $II_a$ et/ou d'un produit répondant à la formule $II_b$ et à réaliser, le cas échéant, une acylation du produit $II_a$ ou une salification ou une estérification du produit $II_b$.

6. Procédé selon la revendication 5, caractérisé en ce que le solvant utilisé pour réaliser l'extraction possède un paramètre de solubilité p' inférieur à 5,5, de préférence compris entre 0,1 et 4,5.

7. Procédé selon la revendication 6, caractérisé en ce que le solvant utilisé pour réaliser l'extraction est choisi parmi le groupe constitué par : le n-pentane, le n-hexane, l'éther de pétrole, le cyclohexane, le n-décane, le dichlorométhane, l'isopropanol, le n-propanol, le chloroforme, l'éthanol, l'acétate d'éthyle, l'acétone et le méthanol.

8. Utilisation d'au moins un extrait de plante du genre Commiphora, en particulier de la plante Commiphora mukul, comme agent cosmétique destiné à modifier la surface de la peau en lui conférant un aspect plus lisse par l'atténuation de la profondeur des rides et des ridules.

9. Utilisation selon la revendication 8, caractérisée en ce que ledit extrait est un extrait de la résine de la plante Commiphora mukul, encore appelée Guggul.

10. Utilisation selon les revendications 8 ou 9, caractérisée en ce que ledit extrait est obtenu après broyat des agrégats de la résine, par extraction par un solvant de polarité p' inférieur à 5,5 , de préférence compris entre 0,1 et 4,5.

11. Utilisation selon la revendication 10, caractérisé en ce que le solvant utilisé pour réaliser l'extraction est choisi parmi le groupe constitué par : le n-pentane, le n-hexane, l'éther de pétrole, le cyclohexane, le n-décane, le dichlorométhane, l'isopropanol, le n-propanol, le chloroforme, l'éthanol, l'acétate d'éthyle, l'acétone et le méthanol.

12. Utilisation selon l'une des revendications 8 ou 9, caractérisée en ce que ledit extrait est obtenu par extraction de la plante Commiphora mukul par le gaz carbonique à l'état supercritique.

**13.** Utilisation selon l'une des revendications 8 ou 12, caractérisée en ce que ledit extrait est enrichi en produit selon l'une des revendications 1 à 4 par fractionnement, notamment par fractionnement par chromatographie liquide haute performance à partir de l'extrait décrit dans la revendication 10 ou 11.

**14.** Utilisation d'au moins un produit tel que défini à l'une quelconque des revendications 1 à 4 comme agent cosmétique destiné à modifier la surface de la peau en lui conférant un aspect plus lisse par l'atténuation de la profondeur des rides et des ridules.

**15.** Procédé de traitement cosmétique destiné à modifier la surface de la peau en lui conférant un aspect plus lisse par l'atténuation de la profondeur des rides et des ridules, caractérisé en ce que l'on applique sur les zones de la peau à traiter, en particulier sur le visage, une composition cosmétique contenant l'un au moins des produits tels que définis dans l'une quelconque des revendications 1 à 4, ou un extrait de plante contenant l'un de ces produits, en particulier un extrait de plante du genre Commiphora, encore en particulier de la plante Commiphora mukul.

**16.** Procédé selon la revendication 15, caractérisé en ce que ladite composition cosmétique contient de 0,001 à 1 % en poids de l'un au moins des produits précités, de préférence de 0,01 à 0,1 %.

**17.** Procédé selon l'une des revendications 15 ou 16, caractérisé en ce que ladite composition cosmétique contient de 0,005 à 5 % en poids, de préférence de 0,05 à 1 % en poids d'un extrait de Commiphora mukul contenant au moins l'un des produits précités.

**18.** Procédé selon la revendication 17, caractérisé en ce que l'extrait de Commiphora mukul est un extrait de résine de cette plante.

**19.** Procédé selon l'une des revendications 15 à 18, caractérisé en ce ladite composition cosmétique contient en outre un produit agissant sur la synthèse de la fibronectine, tel qu'un galactolipide, en particulier un galactosylglycéride.

**20.** Procédé selon l'une des revendications 15 à 19, caractérisé en ce ladite composition cosmétique contient en outre un produit agissant sur la synthèse du collagène, tel que la vitamine C.

**21.** Procédé selon l'une des revendications 15 à 20, caractérisé en ce que ladite composition cosmétique contient un extrait de la plante Commiphora mukul tel que défini dans l'une quelconque des revendications 10 à 13.

**Patentansprüche**

**1.** Produkt, das der Formel (I) entspricht:

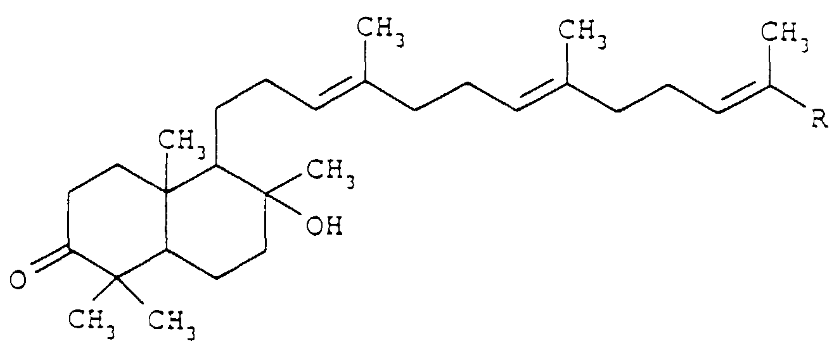

(I)

in der R für:

a) eine Gruppe $CH_2OH$,
b) eine Gruppe COOH

steht, sowie dessen Salze oder Ester.

**2.** Produkt nach Anspruch 1, das der Formel (II) entspricht:

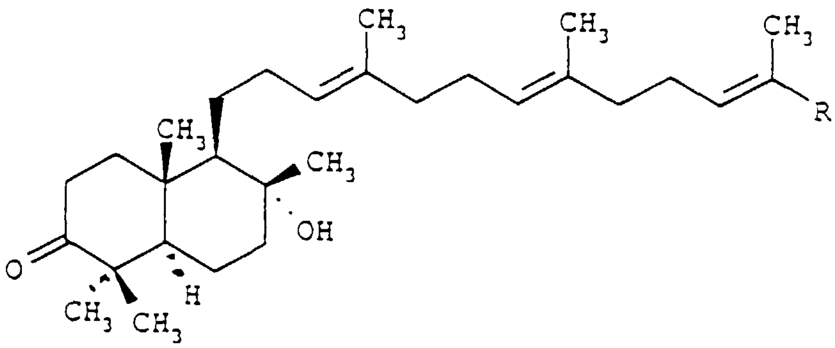

(II)

in der R für:

a) eine Gruppe $CH_2OH$, wobei das Produkt durch die Formel $II_a$ bezeichnet wird,
b) eine Gruppe COOH, wobei das Produkt durch die Formel $II_b$ bezeichnet wird,
c) eine Gruppe

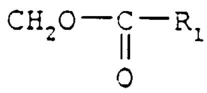

in der $R_1$ für eine lineare oder verzweigte Alkylgruppe, die 1 bis 6 Kohlenstoffatome umfaßt, insbesondere die Methylgruppe, steht,
d) eine Gruppe COOM, worin M ein Alkalimetall, vorzugsweise Natrium oder Kalium, oder eine Ammoniumgruppe oder quartäre Amingruppe bezeichnet,
e) eine Gruppe $COOM'_{0,5}$, worin M' ein Erdalkalimetall, vorzugsweise Calcium, bezeichnet,
f) eine Gruppe $COOR_2$, worin $R_2$ eine lineare oder verzweigte Alkylgruppe, die 1 bis 6 Kohlenstoffatome umfaßt, bezeichnet,

steht.

**3.** Produkt nach Anspruch 2, dadurch gekennzeichnet, daß es der Formel ($II_a$) entspricht:

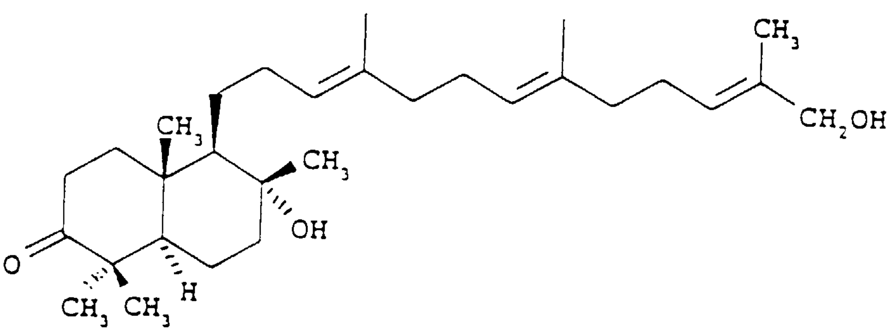

(IIa)

**4.** Produkt nach Anspruch 2, dadurch gekennzeichnet, daß es der Formel ($II_b$) entspricht:

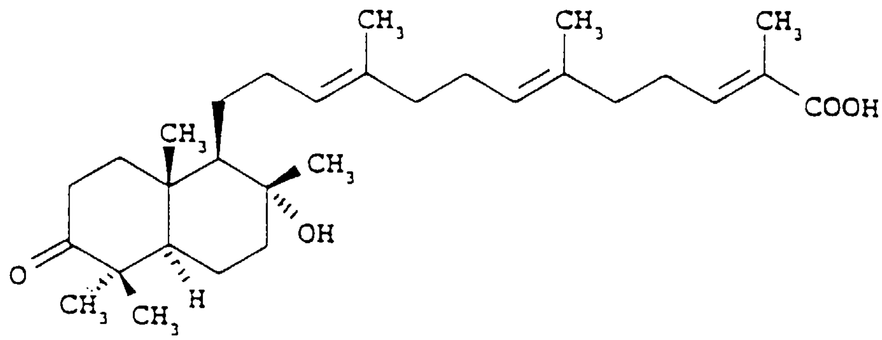

(IIb)

**5.** Verfahren zur Herstellung eines Produkts, wie es in einem der Ansprüche 1 bis 4 definiert worden ist, dadurch gekennzeichnet, daß es darin besteht, Harz von Commiphora mukul durch Extraktion durch ein organisches Lösemittel zu behandeln, um einen als Extrakt G bezeichneten Extrakt herzustellen, und den Extrakt G mindestens einem Fraktionierungsschritt zu unterwerfen, um eine Fraktion zu isolieren, die hauptsächlich aus einem der Formel $II_a$ entsprechenden Produkt und/oder einem der Formel $II_b$ entsprechenden Produkt gebildet wird, und gegebenenfalls eine Acylierung des Produkts $II_a$ oder eine Salzbildung oder eine Veresterung des Produkts $II_b$ vorzunehmen.

**6.** Verfahren nach Anspruch 5, dadurch gekennzeichnet, daß das für die Ausführung der Extraktion verwendete Lösemittel einen Löslichkeitsparameter p' unter 5,5, vorzugsweise zwischen 0,1 und 4,5 aufweist.

**7.** Verfahren nach Anspruch 6, dadurch gekennzeichnet, daß das für die Ausführung der Extraktion verwendete Lösemittel aus der Gruppe, gebildet aus: n-Pentan, n-Hexan, Petrolether, Cyclohexan, n-Decan, Dichlormethan, Isopropanol, n-Propanol, Chloroform, Ethanol, Ethylacetat, Aceton und Methanol, ausgewählt wird.

**8.** Verwendung von mindestens einem Extrakt von Pflanzen der Gattung Commiphora, insbesondere von der Pflanze Commiphora mukul, als kosmetisches Mittel, das dazu bestimmt ist, die Oberfläche der Haut zu modifizieren, indem es ihr ein glatteres Aussehen durch Verminderung der Tiefe der Falten und Fältchen verleiht.

**9.** Verwendung nach Anspruch 8, dadurch gekennzeichnet, daß der Extrakt ein Extrakt des Harzes der Pflanze Commiphora mukul, der auch als Guggul bezeichnet wird, ist.

**10.** Verwendung nach den Ansprüchen 8 oder 9, dadurch gekennzeichnet, daß der Extrakt nach Zerkleinerung der Aggregate des Harzes durch Extraktion durch ein Lösemittel mit einer Polarität p' unter 5,5, vorzugsweise zwischen 0,1 und 4,5, erhalten wird.

**11.** Verwendung nach Anspruch 10, dadurch gekennzeichnet, daß das zur Ausführung der Extraktion verwendete Lösemittel aus der Gruppe, gebildet aus: n-Pentan, n-Hexan, Petrolether, Cyclohexan, n-Decan, Dichlormethan, Isopropanol, n-Propanol, Chloroform, Ethanol, Ethylacetat, Aceton und Methanol, ausgewählt wird.

**12.** Verwendung nach einem der Ansprüche 8 oder 9, dadurch gekennzeichnet, daß der Extrakt durch Extraktion der Pflanze Commiphora mukul durch Kohlensäuregas in überkritischem Zustand erhalten wird.

**13.** Verwendung nach einem der Ansprüche 8 oder 12, dadurch gekennzeichnet, daß der Extrakt an Produkt nach einem der Ansprüche 1 bis 4 durch Fraktionierung, insbesondere durch Fraktionierung mittels Hochleistungsflüssigkeitschromatographie, ausgehend von dem in Anspruch 10 oder 11 beschriebenen Extrakt angereichert ist.

**14.** Verwendung von mindestens einem Produkt, wie es in einem der Ansprüche 1 bis 4 definiert ist, als kosmetisches Mittel, das dazu bestimmt ist, die Oberfläche der Haut zu modifizieren, indem es ihr ein glatteres Aussehen durch Verminderung der Tiefe der Falten und Fältchen verleiht.

**15.** Kosmetisches Behandlungsverfahren, das dazu bestimmt ist, die Oberfläche der Haut zu modifizieren, indem es ihr ein glatteres Aussehen durch Verminderung der Tiefe der Falten und Fältchen verleiht, dadurch gekennzeichnet,

daß man auf die Bereiche der zu behandelnden Haut, insbesondere auf das Gesicht, eine kosmetische Zusammensetzung, die mindestens eines der Produkte, wie sie in einem der Ansprüche 1 bis 4 definiert worden sind, oder einen Pflanzenextrakt, der eines dieser Produkt enthält, insbesondere einen Extrakt von Pflanzen der Gattung Commiphora, noch mehr bevorzugt von der Pflanze Commiphora mukul, aufträgt.

**16.** Verfahren nach Anspruch 15, dadurch gekennzeichnet, daß die kosmetische Zusammensetzung 0,001 bis 1 Gew.-% von mindestens einem der vorstehend erwähnten Produkte, vorzugsweise 0,01 bis 0,1% enthält.

**17.** Verfahren nach einem der Ansprüche 15 oder 16, dadurch gekennzeichnet, daß die kosmetische Zusammensetzung 0,005 bis 5 Gew.-%, vorzugsweise 0,05 bis 1 Gew.-% eines Extrakts von Commiphora mukul, der mindestens eines der vorstehend erwähnten Produkte enthält, enthält.

**18.** Verfahren nach Anspruch 17, dadurch gekennzeichnet, daß der Extrakt von Commiphora mukul ein Extrakt von Harz von dieser Pflanze ist.

**19.** Verfahren nach einem der Ansprüche 15 bis 18, dadurch gekennzeichnet, daß die kosmetische Zusammensetzung darüber hinaus ein Produkt, das auf die Synthese von Fibronectin einwirkt, wie ein Galactolipid, insbesondere ein Galactosylglycerid, enthält.

**20.** Verfahren nach einem der Ansprüche 15 bis 19, dadurch gekennzeichnet, daß die kosmetische Zusammensetzung darüber hinaus ein Produkt, das auf die Synthese von Collagen einwirkt, wie Vitamin C, enthält.

**21.** Verfahren nach einem der Ansprüche 15 bis 20, dadurch gekennzeichnet, daß die kosmetische Zusammensetzung einen Extrakt der Pflanze Commiphora mukul, wie er in einem der Ansprüche 10 bis 13 definiert worden ist, enthält.

**Claims**

**1.** Product of formula (I):

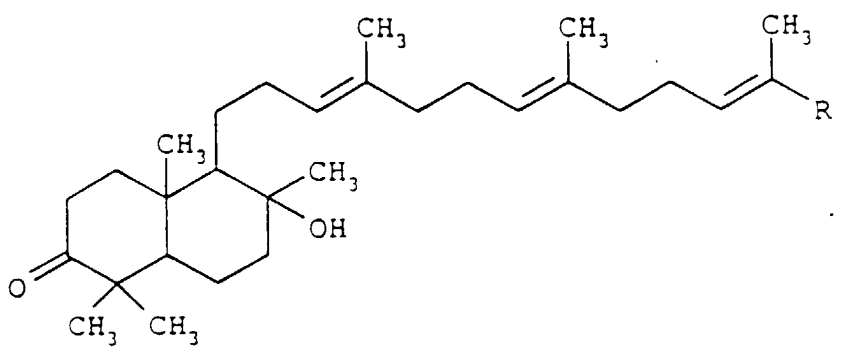

(I)

in which R is:

a) a $CH_2OH$ group or
b) a COOH group,

and the salts or esters thereof.

**2.** Product according to claim I of formula (II):

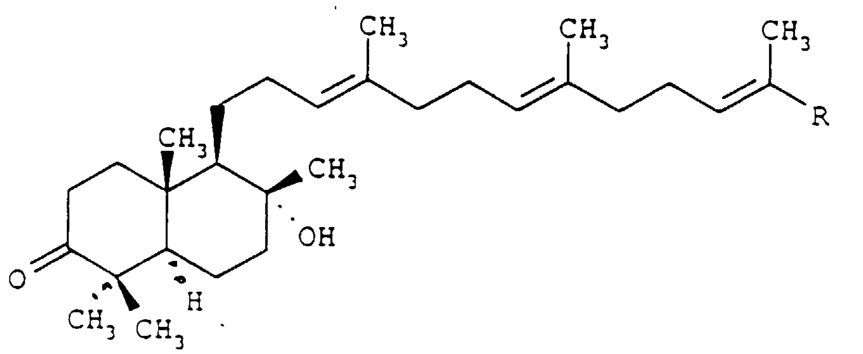

(II)

in which R is:

a) a $CH_2OH$ group, the product being denoted by formula $II_a$,
b) a COOH group, the product being denoted by formula $II_b$,
c) a group

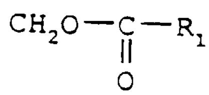

in which $R_1$ is a linear or branched alkyl group comprising from 1 to 6 carbon atoms, particularly the methyl group,
d) a group COOM, where M denotes an alkali metal, preferably sodium or potassium, or an ammonium or quaternary amine group,
e) a group $COOM'_{0.5}$, where M' denotes an alkaline earth metal, preferably calcium, or
f) a group $COOR_2$, where $R_2$ denotes a linear or branched alkyl group comprising from 1 to 6 carbon atoms.

**3.** Product according to claim 2, characterized in that it has formula ($II_a$);

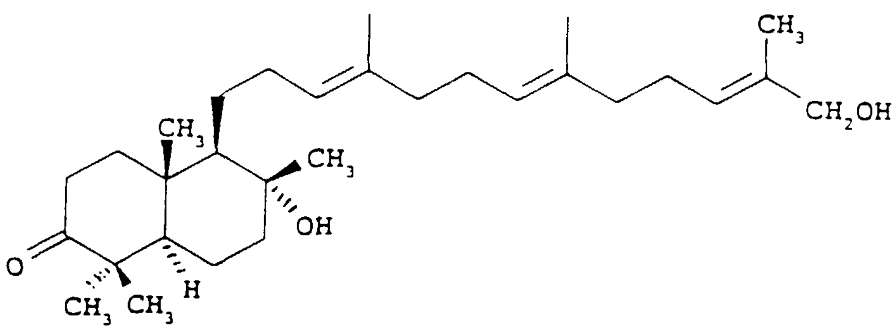

(IIa)

**4.** Product according to claim 2, characterized in that it has formula ($II_b$):

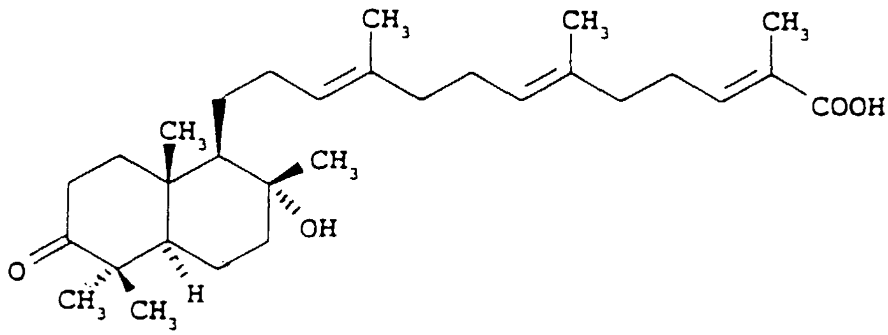

(IIb)

5. Process for the preparation of a product as defined in one of claims I to 4, characterized in that it consists in treating Commiphora mukul resin by extraction with an organic solvent in order to prepare an extract, called extract G, subjecting said extract G to at least one fractionation step in order to isolate a fraction consisting predominantly of a product of formula $II_a$ and/or a product of formula $II_b$, and, if appropriate, acylating the product $II_a$ or salifying or esterifying the product $II_b$.

6. Process according to claim 5, characterized in that the solvent used to perform the extraction has a solubility parameter p' of less than 5.5, preferably of between 0.1 and 4.5.

7. Process according to claim 6, characterized in that the solvent used to perform the extraction is selected from the group consisting of n-pentane, n-hexane, petroleum ether, cyclohexane, n-decane, dichloromethane, isopropanol, n-propanol, chloroform, ethanol, ethyl acetate, acetone and methanol.

8. Use of at least one extract of a plant of the genus Commiphora, particularly of the Commiphora mukul plant, as a cosmetic agent for modifying the surface of the skin to give it a smoother appearance by reducing the depth of the large and small wrinkles.

9. Use according to claim 8, characterized in that said extract is an extract of the resin of the Commiphora mukul plant, which is also called guggul.

10. Use according to claim 8 or 9, characterized in that said extract is obtained, after grinding of the resin aggregates, by extraction with a solvent whose polarity p' is less than 5.5, preferably between 0.1 and 4.5.

11. Use according to claim 10, characterized in that the solvent used to perform the extraction is selected from the group consisting of n-pentane, n-hexane, petroleum ether, cyclohexane, n-decane, dichloromethane, isopropanol, n-propanol, chloroform, ethanol, ethyl acetate, acetone and methanol.

12. Use according to claim 8 or 9, characterized in that said extract is obtained by extraction of the Commiphora mukul plant with gaseous carbon dioxide in the supercritical state.

13. Use according to claim 8 or 12, characterized in that said extract is enriched in a product according to one of claims I to 4 by fractionation, especially by high performance liquid chromatography, of the extract described in claim 10 or 11.

14. Use of at least one product as defined in any one of claims I to 4 as a cosmetic agent for modifying the surface of the skin to give it a smoother appearance by reducing the depth of the large and small wrinkles.

15. Method of cosmetic treatment for modifying the surface of the skin to give it a smoother appearance by reducing the depth of the large and small wrinkles, characterized in that a cosmetic composition containing at least one of the products as defined in any one of claims I to 4, or a plant extract containing one of these products, particularly an extract of a plant of the genus Commiphora and very particularly of the Commiphora mukul plant, is applied to the areas of skin to be treated, particularly on the face.

16. Method according to claim 15, characterized in that said cosmetic composition contains from 0.001 to 1% by weight,

preferably from 0.01 to 0.1%, of at least one of the products mentioned above.

**17.** Method according to claim 15 or 16, characterized in that said cosmetic composition contains from 0.005 to 5% by weight, preferably from 0.05 to 1% by weight, of a Commiphora mukul extract containing at least one of the products mentioned above.

**18.** Method according to claim 17, characterized in that the Commiphora mukul extract is an extract of the resin of this plant.

**19.** Method according to one of claims 15 to 18, characterized in that said cosmetic composition also contains a product which acts on fibronectin synthesis, such as a galactolipid, particularly a galactosylglyceride.

**20.** Method according to one of claims 15 to 19, characterized in that said cosmetic composition also contains a product which acts on collagen synthesis, such as vitamin C.

**21.** Method according to one of claims 15 to 20, characterized in that said cosmetic composition contains an extract of the Commiphora mukul plant as defined in any one of claims 10 to 13.

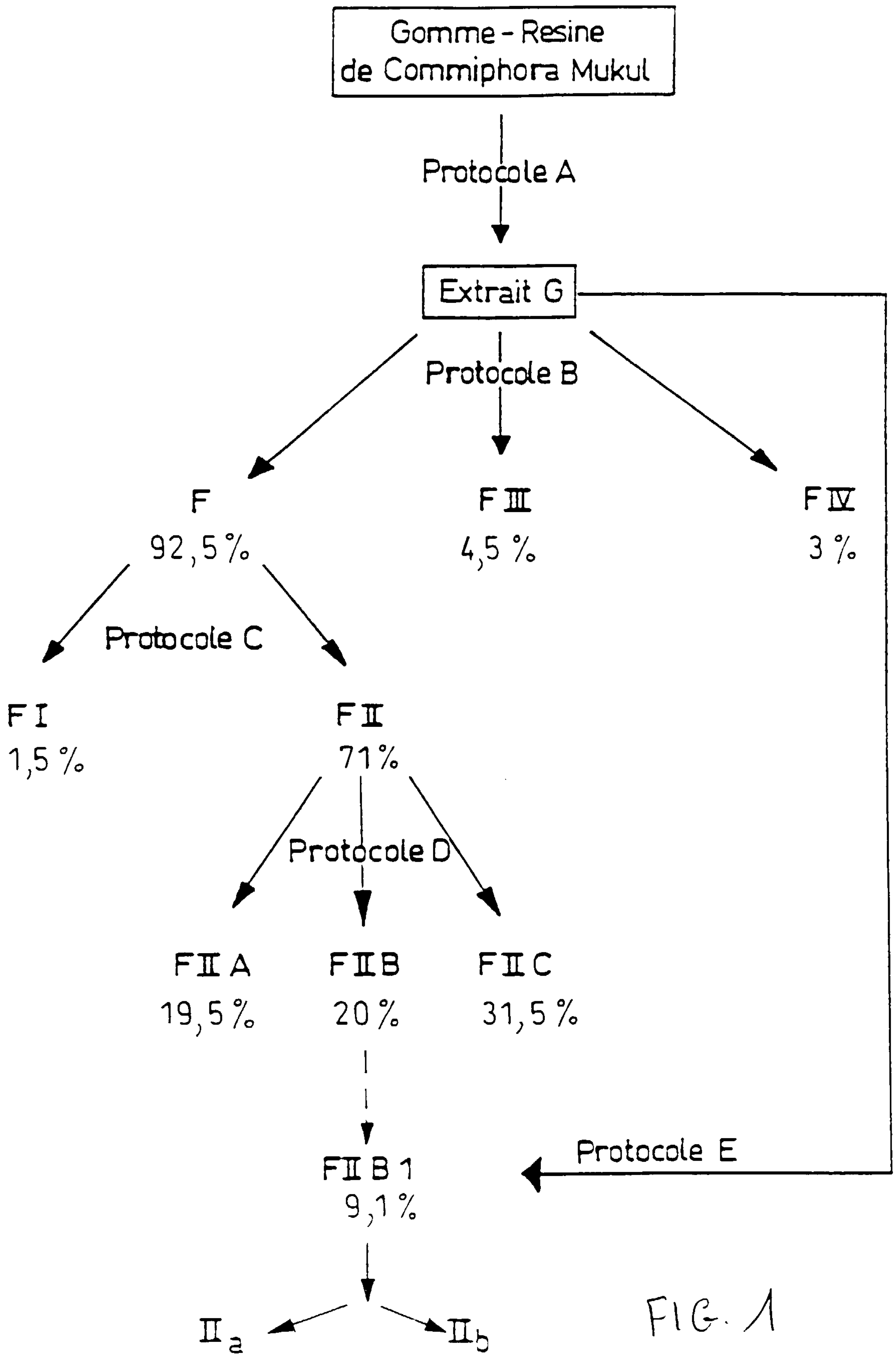
Gomme - Resine
de Commiphora Mukul
Protocole A
Extrait G
Protocole B
F
92,5 %
F III
4,5 %
F IV
3 %
Protocole C
F I
1,5 %
F II
71 %
Protocole D
F II A
19,5 %
F II B
20 %
F II C
31,5 %
F II B 1
9,1 %
Protocole E
II a
II b

FIG. 1